# EUROPÄISCHE PATENTSCHRIFT

(11) **EP 1 367 946 B1**
(45) Veröffentlichungstag und Bekanntmachung des Hinweises auf die Patenterteilung: **17.10.2007**
(21) Anmeldenummer: 02712921.2
(22) Anmeldetag: 20.02.2002
(51) Int. Cl.: A61B 17/15

(54) **SYSTEM ZUR REKONSTRUKTION DER NATÜRLICHEN TORQUIERTHEIT ZWISCHEN DEM NATÜRLICHEN KNIE UND DEM BEREICH DER NATÜRLICHEN HÜFTE**
SYSTEM FOR RECONSTRUCTING THE NATURAL TORSION BETWEEN THE NATURAL KNEE AND THE NATURAL HIP AREA
SYSTEME POUR RETABLIR LA FACULTE DE TORSION NATURELLE ENTRE UN GENOU NATUREL ET LA ZONE D'UNE HANCHE NATURELLE

(30) Priorität: 15.03.2001 DE 10113069
(43) Veröffentlichungstag der Anmeldung: 10.12.2003
(73) Patentinhaber: ESKA Implants GmbH & Co. KG, 23556 Lübeck (DE)
(72) Erfinder: GRUNDEI, Hans, 23558 Lübeck (DE)
(74) Vertreter: Fuchs
(86) Internationale Anmeldenummer: PCT/EP2002/001771
(87) Internationale Veröffentlichungsnummer: WO 2002/074172

(56) Entgegenhaltungen:
- DE-C- 19 716 300
- DE-C- 19 753 236
- FR-A- 2 679 766
- FR-A- 2 726 178
- US-A- 5 649 928
- US-A- 5 658 293

## Beschreibung

Die vorliegende Erfindung betrifft ein System zur Rekonstruktion der natürlichen Torquiertheit zwischen dem natürlichen Knie und dem Bereich der natürlichen Hüfte in einer Anordnung zwischen einem künstlichen Kniegelenk und dem Bereich der natürlichen Hüfte nach teilweiser Resektion der natürlichen Kondylen des Kniegelenks in einem Horizontalschnitt.

Aus der DE-197 16 300 C1 ist ein derartiges System bekannt. Es verfügt über eine Nagellehre mit einem exakt quaderförmigen Anlageblock, der an seiner von ventral gesehenen Stirnfläche einen davon abragenden, femurwärts weisenden Schenkel, der an seinem Ende einen Femurkontaktfühler in Form eines senkrecht auf dem Schenkel stehenden Bolzens zur ventral gelegenen punktförmigen Auflage auf dem Femur hält, und an seiner von dorsal gesehenen Stirnfläche mindestens eine davon abragende, femurwärts weisende Anschlagslasche zur jeweils dorsal gelegenen punktförmigen Anlage an beiden dorsalen Kondylenrollen aufweist. Der Anlageblock ist dabei durch Bohrungspaare durchsetzt, deren Anordnung die jeweilige Größe des Femurteils repräsentiert, durch welche Fixationsnägel setzbar sind, welche den Anlageblock in seiner Anlage auf dem Femur sichert. Der Anlageblock ist dabei unter Zurückbelassung der Fixationsnägel vom Femur abziehbar. Darüber hinaus sieht das System eine Sägelehre mit identischer Grundform wie der Anlageblock der Nagellehre und mit identischer Anordnung der Bohrungspaare in dem Anlageblock vor. Die Sägelehre ist auf die Fixationsnägel setzbar, welche mit Hilfe der Nagellehre gesetzt wurden. Dabei legen ihre Stirnseiten die übrigen Resektionsebenen (ventral, dorsal, diagonal) fest.

Eine Weiterbildung dieser Resektionslehre ist bekannt aus der DE-197 53 236 C1. Dieses System hat es sich zur Aufgabe gemacht, eine exakte Nachbildung der natürlichen Torquiertheit zwischen dem Knie und der Hüfte abzubilden unter der Voraussetzung, daß der Resektionsschnitt an der Tibia kein Horizontalschnitt, sondern vielmehr ein geneigter Schnitt ist, der in einem Winkel zwischen 3° bis 5° gegenüber der Horizontalen von lateral nach medial geneigt ist. Hierzu weist dieses System gegenüber dem vorerwähnten System einen Keil mit einem Keilwinkel auf, der zwischen den lateralen Kondylenrollen und der Anschlagslasche schiebbar ist zur Kompensation eines Keilwinkels, in dem der horizontale Tibiaschnitt von der Kniespaltachse abweicht.

Dem vorerwähnten System haftet der Nachteil an, daß in der Praxis der Operateur geneigt ist, stets denselben Keil mit demselben Keilwinkel anzusetzen. Dies wird jedoch nicht allen patientenindividuellen Anforderungen gerecht.

Vor diesem Hintergrund ist es die Aufgabe der vorliegenden Erfindung, das erwähnte System so weiterzubilden, daß es vielseitiger einsetzbar ist und vor allem den jeweiligen Gegebenheit angepaßt werden kann.

Gelöst wird diese Aufgabe mit einem System mit den Merkmalen des Anspruchs 1. Vorteilhafte Weiterbildungen ergeben sich aus den Unteransprüchen.

Ausgehend von dem eingangs erwähnten System weist das erfindungsgemäße System die nachfolgend aufgeführten Komponenten auf:
eine Sägelehre in Form eines exakten Quaders, der durchsetzt ist mit Sägeschlitzen zur Erzeugung von senkrechten Schnitten ventral und dorsal sowie der Diagonalschnitte, die mit einer Frontalseite auf den Horizontalschnitt des Femurs setzbar und an diesem lösbar fixierbar ist, die eine zentrale Durchbrechung aufweist und mit Kopplungsmitteln versehen ist;
eine Rotationslehre, an welche die Sägelehre (2) über die Kopplungsmittel lösbar ankoppelbar ist und welche die Sägelehre dabei übergreift und mindestens eine abragende, femurwärts weisende Anschlagslasche zur jeweils dorsal gelegenen punkförmigen Anlage an beiden dorsalen Femurkondylenrollen aufweist;
einen femurseitig mit der Rotationslehre durch die Durchbrechung hindurch verschwenkbar verbindbaren intramedullären Spieß zum Einsatz im fernoralen Knochenkanal, wobei die Sägelehre auf dem intramedullären Spieß auffädelbar und dann mit der Rotationslehre koppelbar ist und wobei der intramedulläre Spieß eine Verschwenkachse für' die Sägelehre bildet, urn die herum diese in einem Bereich von ± 10° um die Horizontale verschwenkbar ist;
einen Getriebemechanismus, welcher die Sägelehre um die von dem intramedullären Spieß vorgegebene Verschwenkachse in dem Horizontalabschnitt (H) des Femurs herum verschwenkt;
einen mit der Funktionseinheit aus Sägelehre und Rotationslehre lösbar verbindbaren und femurwärts weisenden Schenkel, der an seinem Ende einen ventralen Femurkontaktfühler in Form eines senkrecht auf dem Schenkel stehenden Bolzens zur ventral gelegenen punktförmigen Auflage auf dem Femur aufweist.

Mit dem erfindungsgemäßen System ist es möglich, ohne Verwendung einer Nagellehre aus dem Stand der Technik die Sägelehre so am Horizontalschnitt des Femurs auszurichten und zu fixieren, daß keinerlei Wechsel mehr zwischen einer Nagellehre und Sägelehre stattfindet. Vielmehr gestattet das erfindungsgemäße System die stufenlose Einstellung der Sägelehre im Bereich von ± 10° gegenüber der Horizontalen dank der Rotationslehre und die anschließende Fixation der Sägelehre mit wenigstens zwei Fixationsnägeln am Femur. Sodann kann die Rotationslehre von der Sägelehre abgekoppelt werden, ebenso wie der Femurkontaktfühler entfernt werden kann. Zur Erzeugung von den senkrechten Schnitten ventral und dorsal sowie der Diagonalschnitte wird die Sägelehre gemäß einer vorteilhaften Weiterbildung mittels zusätzlicher Befestigungsmittel zur temporären Fixation am Femur befestigt. Ein zusätzliches Befestigungsmittel können zwei seitliche Laschen sein, durch welche Bohrungen greifen, durch welche Knochenschrauben führbar und mit dem Knochen des Femurs verschraubbar sind.

Von wesentlicher Bedeutung bei dem erfindungsgemäßen System ist, daß die Rotationslehre, auf welche die Sägelehre zunächst aufgefädelt ist, auf dem femurseitig mit der Rotationslehre verschwenkbar verbundenen intramedullären Spieß in eine stabile Dreipunktanlage am Femur gebracht wird. Die erwähnten Berührungspunkte der Anschlagslasche der Rotationslehre an den hinteren Kondylenrollen bilden zwei Punkte für diese anvisierte stabile Dreipunktanlage. Der dritte Punkt hierfür ist der Berührungspunkt des Femurkontaktfühlers der Funktionseinheit aus Sägelehre und Rotationslehre. Dies bedeutet, daß der Femurkontaktfühler mit der Sägelehre, oder mit der Rotationslehre lösbar verbindbar ist. Da in diesem Zustand die Sägelehre mit der Rotationslehre verkoppelt ist, sind beide Ansätze denkbar.

Der intramedulläre Spieß definiert mit seiner formschlüssigen Anlage im intramedullären Knochenkanal die Verschwenkachse für die Sägelehre. Zusammen mit dem ventralen Femurkontakrfühler bildet er ein für den jeweiligen Femur eindeutig definiertes Bezugssystem, in welchem die natürliche Torquiertheit abgebildet ist. Der intramedulläre Spieß ist die Voraussetzung für die weitere Komponente, nämlich dem Getriebemechanismus, mit dessen Hilfe die Sägelehre um die Verschwenkachse, welche durch den intramedullären Spieß definiert ist, verschwenkt werden kann. Die Verschwenkung ist die Verallgemeinerung des Prinzips, welches aus der DE 197 53 236 C1 bekannt ist, bei dem zur Kompensation eines Keilwinkels, in dem der horizontale Tibiaschnitt von der Kniespaltachse abweicht, der oben erwähnte Keil mit vorgegebenem Keilwinkel zum Einsatz kommt. Gegenüber diesem System ist das erfindungsgemäße System äußerst flexibel und gestattet individuelle Einstellungen in einem weiten Bereich von ± 10° um die Horizontale. Hierdurch sind Kompensationen jedweder Mißbildungen und Schäden möglich. Selbst Verschwenkungen in einer Richtung, die entgegengesetzt zu jener ist, wie sie in der DE 197 53 236 C1 beschrieben ist, ist möglich.

Bevorzugt stellt der Operateur den Kompensationswinkel mit dem Getriebemechanismus vor der Resektion ein und führt den intramedullären Spieß dann in den eröffneten Femurkanal. Aufgrund der freien Verschwenkbarkeit des Spießes an der Rotationslehre findet dieser in dem Knochenkanal seine optimale Lage automatisch.

Gemäß einer bevorzugten Weiterbildung ist der intramedulläre Spieß an die Rotationslehre mittels eines Kegelpaßsitzes angelenkt. Dieser gestattet zum einen die schon erwähnte freie Verschwenkbarkeit des Spießes gegenüber der Rotationslehre. Zum anderen gestattet der Kegelpaßsitz ein Austauschen des intramedullären Spießes durch einen Spieß anderer Dimension. Grundsätzlich muß nämlich der intramedullär Spieß patientenindividuell ausgewählt und mittels des Kegelpaßsitzes aufgesteckt werden. Insbesondere der Durchmesser des Spießes spielt hierbei eine wesentliche Rolle.

Bevorzugt ist der Getriebemechanismus der Rotationslehre zur Einstellung des Kompensationswinkels stufenlos verstellbar. Dies hat unmittelbar die stufenlose Einstellbarkeit des Kompensationswinkels in dem angegebenen Bereich von ± 10° der Sägelehre von der Horizontalen zur Folge.

Bevorzugt wird, wenn der Getriebemechanismus trotz stufenloser Verstellbarkeit in vorgegebenen Einstellpositionen mechanisch einrastet. Dies erhöht die taktile Wahrnehmung des Operateurs, um welchen Winkel die Sägelehre bereits verschwenkt worden ist. Beispielsweise kann mit jedem zusätzlichen halben Grad Zunahme des Verschwenkwinkels ein Einrasten oder Klicken im Getriebemechanismus erzeugt werden, so daß der Operateur vor allem vor dem Hintergrund der äußerst beengten räumlichen Verhältnisse während der Operation eine Vorstellung davon erhält, um welchen Winkel die Sägelehre bereits verschwenkt worden ist.

Die Erfindung wird anhand eines Ausführungsbeipspiels näher erläutert. Hierbei zeigt:
- Figur 1: die Frontalansicht der Rotationslehre mit angekoppelter Sägelehre,
- Figur 2: die perspektivische Ansicht der Funktionseinheit aus Rotationslehre und Sägelehre,
- Figur 3: die Sägelehre, abgekoppelt von der Rotationslehre, und
- Figur 4: schematisch die Anlage der Rotationslehre mit angekoppelter Sägelehre an einem Femurknochen mit bereits erfolgter horizontaler Resektion.

Nachfolgend sind gleiche Teile mit denselben Bezugszeichen versehen.

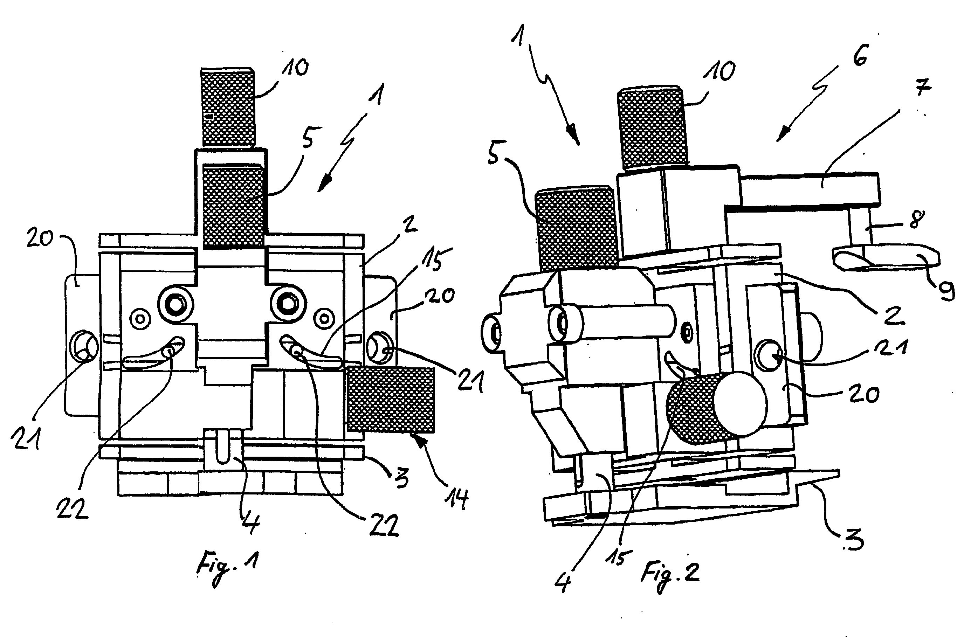

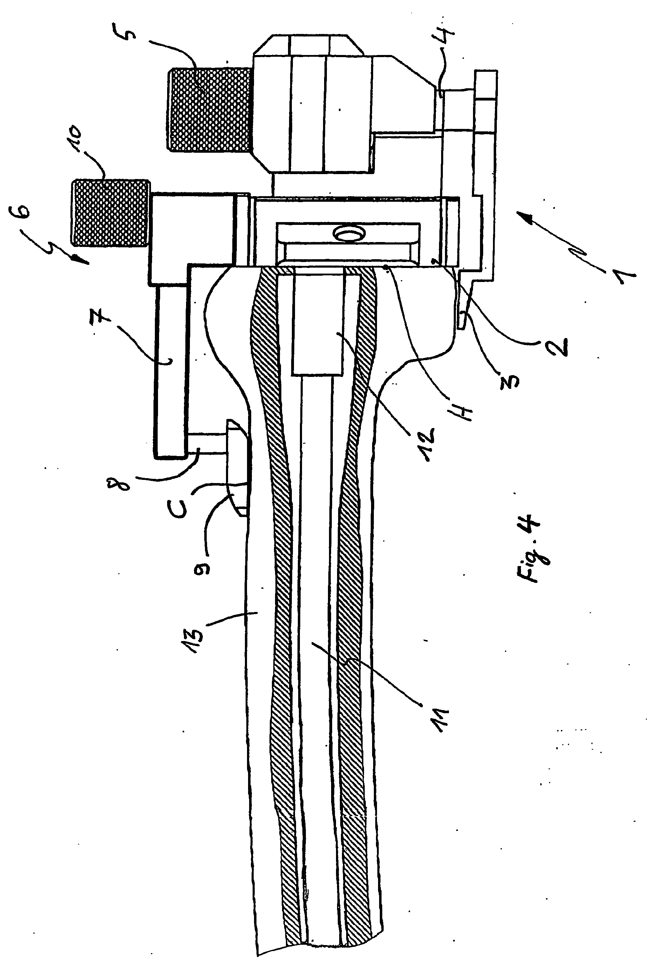

Figur 1 zeigt die Frontalansicht der Rotationslehre 1 des erfindungsgemäßen Systems mit daran angekoppelter Sägelehre 2. Die Rotationslehre 1 weist an der dorsal gelegenen Seite (in Figur 1 also unten) eine Anschlagslasche 3 auf, welche mit den hinteren Kondylenrollen des Femurs lediglich an zwei Punkten in Berührung kommt (Figur 4). Vorliegend ist die Anschlagslasche 3 mit der Rotationslehre über ein Getriebegestänge 4 verbunden, welches mit einer Rändelschraube 5 als Getriebemechanismus so zusammenarbeitet, daß der Abstand der Anschlagslasche 3 von den Kondylen variiert werden kann, derart, daß die Rotationslehre nach Art einer Schiebelehre zur Größenbestimmung- und Festlegung des Femurteils des künstlichen Kniegelenkes dient. Der Operateur muß nach Festlegung der Größe des Femurteilimplantates die Rotationslehre in eine stabile Dreipunktauflage mit den beiden Auflagepunkten an den hinteren Kondylenrollen und Berührungspunkt C bringen. Erst dann ist der Bezug hergestellt zur Torquiertheit zwischen dem Knie- und dem Hüftbereich des Patienten. Die erwähnten Berührungspunkte der Anschlagslasche 3 an den hinteren Kondylenrollen bilden zwei Punkte für eine anvisierte stabile Dreipunktlage. Der dritte Punkt hierfür ist der Berührungspunkt C (Figur 4) eines Femurkontaktfühlers 6 des Systems. Der Femurkontaktfühler 6 ist vorliegend ausgebildet als ein senkrecht auf einem von der ventral gelegenen Stirnseite der Sägelehre 2 abragenden, femurwärts weisenden Schenkel 7 stehender Bolzen 8 mit daran befestigtem Auflageschuh 9.

Eine Schraube 10, mit welcher der Femurkontaktfühler an der Sägelehre 2 angebracht ist, ermöglicht das Heranführen des Femurkontaktfühlers 6 an den Femur von ventral.

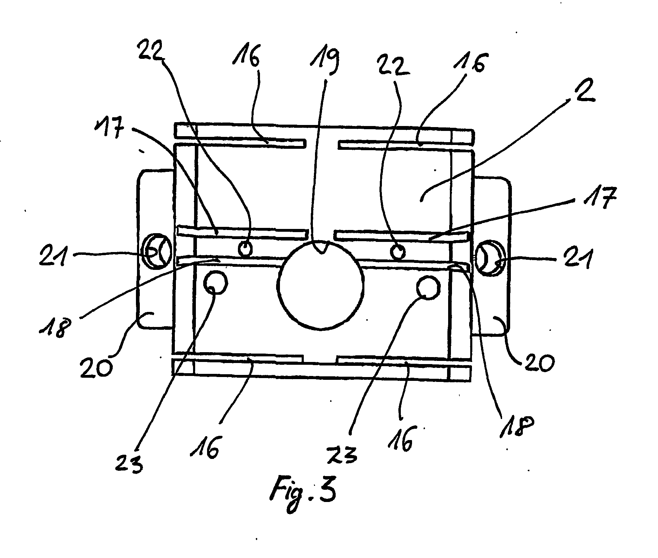

Einzelheiten der Sägelehre 2 ergeben sich aus Figur 3. Die Sägelehre 2 ist als exakter Quader ausgeführt und ist durchsetzt durch Sägeschlitze 16, 17 und 18. Die Sägeschlitze 16 dienen zur Erzeugung der senkrechten Schnitte ventral und dorsal, wohingegen die Sägeschlitze 17 und 18 zur Erzeugung der Diagonalschnitte dienen. Seitlich, d.h. lateral und medial, sind Laschen 20 angeformt, die jeweils durch eine Durchbohrung 21 durchsetzt sind. Diese dienen zum Durchgreifen von Knochenschrauben (nicht dargestellt) für die temporäre Fixation der in ihrer Lage richtig eingestellten Sägelehre 2 zur Vorbereitung der Resektionen, damit die Lage der Sägelehre 2 auch während der Resektionsschnitte unverändert lagetreu bleibt.

Die Sägelehre 2 weist eine zentrale Durchbrechung 19 auf, durch welche der intramedulläre Spieß 11 hindurchgreift, wenn das erfindungsgemäße System zum Einsatz kommt.

Durchbohrungen 22 in der Sägelehre 2 dienen zum Hindurchführen von Fixationsnägeln (nicht dargestellt), nachdem die Winkellage der Sägelehre 2 bezüglich der Horizontalen durch Verschwenkung gegenüber der Horizontalen in die richtige Lage gebracht worden ist.

Durchbrechungen 23 oder Sackbohrungen 23 dienen zur temporären Verkoppelung zwischen Sägelehre 2 und der Rotationslehre 1.

Als weiteres wesentliches Element ist in Figur 4 der intramedulläre Spieß 11 dargestellt. Dieser ist mit der Rotationslehre 1 über einen Kegelpaßsitz lösbar verbunden, kann also in allen Richtungen verschwenkt werden. Beim Eröffnen des Knochenkanals des Femurs 13 sucht sich der intramedulläre Spieß 11 gewissermaßen die richtige Lage aus, indem die Form des Knochenkanals die Verschwenkung des Spießes 11 beim Hineinführen in den Knochenkanal vorgibt. Dabei muß der Operateur auf die richtige Auswahl des geeigneten Spießes achten, derart, daß dieser Spieß frei im Knochenkanal zu liegen kommt.

Damit ist das Koordinatensystem für die Anbringung der Resektionsschritte vollständig. Der Operateur muß nun noch die Abweichung der Sägelehre 2 von der Horizontalen festlegen, um eine Kompensation für jedwede Mißbildung oder Schäden durchzuführen, aber auch für eine Kompensation für jenen Winkel sorgen, in welchem der horizontale Tibiaschnitt von der Kniespaltachse abweicht. Hierzu ist ein Schwenkmechanismus 14 (Fig. 1) vorgesehen, mit dessen Hilfe er die Sägelehre 2 aus der Horizontalen um einen Winkel von ± 10° herausschwenken kann. Dies wird anhand der gekrümmten Verläufe der Langöffnungen 15 angedeutet. Die Sägelehre 2 wird durch Betätigung des Getriebemechanismus 14 um eine Achse verschwenkt, welche von dem intramedullären Spieß 11 vorgegeben ist.

Nach Erreichen der gewünschten Position wird die Sägelehre 2 in bekannter Weise an dem Horizontalschnitt des Femurs 13 mit Fixationsnägeln fixiert, die durch die Durchbohrungen 22 in den Femur geschlagen werden.

Die Rotationslehre wird sodann von der Sägelehre 2 abgekoppelt. Danach wird die Sägelehre 2 mit Knochenschrauben, die durch die Durchbohrungen 21 in den Laschen 20 gesetzt werden, am Femur temporär fixiert, um, wie bereits erwähnt, die senkrechten Schnitte ventral und dorsal sowie die Diagonalschnitte durchzuführen, wobei die Torquiertheit aufgrund der Anwendung des erfindungsgemäßen System erhalten bleibt.

Die Anwendung des erfindungsgemäßen Systems wird nachfolgend kurz beschrieben:
Zu Beginn der Resektion wird zunächst die horizontale Anlagefläche H (Figur 4) hergestellt. Vorliegend wird dann die Rotationslehre 1 mit daran angekoppelter Sägelehre 2, die auf dem intramedullären Spieß aufgefädelt ist, an die horizontale Anlagefläche H herangeführt unter gleichzeitiger Einführung des intramedullären Spießes 11 in den Knochenkanal des Femurs 13. Die Rotationslehre 1 ist dabei mit dem Getriebemechanismus 4 und 5 ausgestattet, so daß sie nach Art einer Schiebelehre zur Größenbestimmung des Femurteils des künstlichen Kniegelenkes herangezogen werden kann. Dazu wird die Rotationslehre 1 in die erwähnte stabile Dreipunktanlage gebracht, so daß also die Anschlagslasche 3 die hinteren Kondylenrollen dorsal jeweils nur an einer Stelle berührt, ebenso wie der Femurkontaktfühler 6 den Femur 13 am Punkt C auf der gegenüberliegenden Seite des Femurs. Die stabile Dreipunktanlage wird nicht beeinträchtigt durch einen direkten Kontakt der Sägelehre 2 mit der horizontalen Anlagefläche H. Sodann findet die Verschwenkung der Sägelehre 2 mit Hilfe des Getriebemechanismus 14, 15 in gewünschter Richtung statt, wonach die Sägelehre 2 dann - wie beschrieben - mit Fixationsnägeln am Femur fixiert wird, wonach die Rotationslehre 1 von der Sägelehre 2 abgekoppelt wird, und ebenso wie der Femurkontaktfühler 6 entfernt. Die Sägelehre 2 wird dann mit Knochenschrauben am Femur fixiert und die Resektionsschnitte werden ausgeführt.

## Patentansprüche

1. System zur Rekonstruktion der natürlichen Torquiertheit zwischen dem natürlichen Kniegelenk und dem Bereich der natürlichen Hüfte in einer Anordnung zwischen einem künstlichen Kniegelenk und dem Bereich der natürlichen Hüfte nach teilweiser Resektion der natürlichen Kondylen des Kniegelenks in einem Horizontalschnitt, aufweisend:
- eine Sägelehre (2) in Form eines exakten Quaders, der durchsetzt ist mit Sägeschlitzen (16, 17, 18) zur Erzeugung von senkrechten Schnitten ventral und dorsal sowie der Diagonalschnitte, die mit einer Frontalseite auf den Horizontalschnitt des Femurs (13) setzbar und an diesem lösbar fixierbar ist, die eine zentrale Durchbrechung (19) aufweist und mit Koppelungsmitteln versehen ist
- eine Rotationslehre (1), an welche die Sägelehre (2) über die Kopplungsmittel lösbar ankoppelbar ist und welche die Sägelehre (2) dabei übergreift und mindestens eine abragende, femurwärts weisende Anschlagslasche (3) zur jeweils dorsal gelegenen punktförmigen Anlage an beiden dorsalen Femurkondylenrollen aufweist,
- einen femurseitig mit der Rotationslehre (1) durch die Durchbrechung (19) hindurch verschwenkbar verbindbaren intramedullären Spieß (11) zum Einsatz im femoralen Knochenkanal, wobei die Sägelehre (2) auf dem intramedullären Spieß (11) auffädelbar und dann mit der Rotationslehre (1) koppelbar ist und wobei der intramedulläre Spieß (11) eine Verschwenkachse für die Sägelehre (2) bildet, um die herum diese in einem Bereich von + 10° um die Horizontale verschwenkbar ist,
einen Getriebemechanismus (14), welcher die Sägelehre (2) um die von dem intramedullären Spieß (11) vorgegebene Verschwenkachse in dem Horizotalabschnitt (H) des Femurs herum verschwenkt, und
- einen mit der Funktionseinheit aus Sägelehre (2) und Rotationslehre (1) lösbar verbindbaren femurwärts weisenden Schenkel (7), der an seinem Ende einen ventralen Femurkontaktfühler (6) in Form eines senkrecht auf dem Schenkel (7) stehenden Bolzens (8) zur ventral gelegenen Auflage auf dem Femur (13) aufweist.

2. System nach Anspruch 1, bei dem der intramedulläre Spieß (1) an der Rotationslehre (1) mittels eines Kugelpaßsitzes angelenkt ist.

3. System nach Anspruch 1 oder 2, bei dem der Getriebemechanismus (14) der Rotationslehre stufenlos verstellbar ist.

4. System nach Anspruch 3, bei der Getriebemechanismus (14) in vorgegebenen Einstellpositionen einrastet.

5. System nach einem der Ansprüche 1 bis 4, bei dem die Sägelehre (2) zusätzliche Befestigungsmittel (20) zur temporären Fixation am Femur aufweist.

## Claims

1. A system for reconstructing the natural torsion between the natural knee joint and the region of the natural hip in an arrangement between an artificial knee joint and the region of the natural hip following partial resection of the natural condyles of the knee joint in a horizontal cut, comprising:
- a saw jig (2) in the form of an exact rectangular parallelepiped which is penetrated by saw slits (16, 17, 18) for production of perpendicular cuts ventrally and dorsally, as well as diagonal cuts, said saw jig being placeable with a front side against the horizontal cut of the femur (13) and detachably fixed thereon, and having a central opening (19) and being provided with coupling means,
- a rotational jig (1) to which the saw jig (2) is detachably couplable and which grasps the saw jig (2) and has at least one stopper plate (3) which protrudes towards the femur for dorsally situated point seating on each of the two dorsal femoral condyle rolls,
- an intramedullary spike (11) for insertion into the femoral bone canal, said intramedullary spike being pivotably connectable to the rotational jig (1) on the femur side through the opening (19), wherein the saw jig (2) is threadable on the intramedullary spike (11) and then couplable to the rotational jig (1) and wherein the intramedullary spike (11) forms a pivot axis for the saw jig (2) about which axis said saw jig is pivotable within a range of ± 10°about the horizontal,
- a gearing mechanism (14) which pivots the saw jig (2) about the pivot axis defined by the intramedullary spike (11) in the horizontal section (H) of the femur, and
- a limb (7) pointing towards the femur and being detachably connectable to the functional unit comprising the saw jig (2) and the rotational jig (1), said limb having on its end a ventral femur contact probe (6) in the form of a pin (8) standing perpendicularly on the limb (7), for ventrally situated contact on the femur (13).

2. The system according to claim 1, wherein the intramedullary spike (11) is linked to the rotational jig (1) by means of a ball-fit seating.

3. The system according to claim 1 or 2, wherein the gearing mechanism (14) of the rotational jig (1) is continuously adjustable.

4. The system according to claim 3, wherein said gearing drive mechanism (14) snaps into predetermined adjustment positions.

5. The system according to one of the claims 1 to 4, wherein the saw jig (2) has additional fastening means (20) for temporary fixing on the femur.

## Revendications

1. Système pour rétablir la faculté de torsion naturelle entre l'articulation du genou naturel et la zone de la hanche naturelle dans un arrangement entre une articulation du genou naturel et la zone d'une hanche naturelle après résection partielle des condyles naturels de l'articulation du genou dans une coupe horizontale, présentant :
- une jauge de scie (2) sous forme de parallélépipède exact qui est entrecoupé de fentes de scie (15, 17, 18) pour la génération de coupes verticales ventrales et dorsales ainsi que des coupes diagonales, jauge qui peut être posée avec un côté frontal sur la coupe horizontale du fémur (13) et fixée de manière amovible sur ce dernier, jauge qui présente une ouverture centrale (19) et est dotée de moyens de couplage,
- une jauge de rotation (1) à laquelle la jauge de scie (2) peut être couplée de manière amovible à l'aide des moyens de couplages et qui prend alors par-dessus la jauge de scie (2) et présente au moins un collier de butée (3) saillant orientée vers le fémur vers l'installation ponctuelle chaque fois dorsale sur les deux rouleaux de condyles fémoraux dorsaux,
- une broche intramédullaire (11) pouvant être reliée de manière pivotante côté fémur à travers l'ouverture (19) avec la jauge de rotation (1) pour placement dans le canal osseux fémoral, étant donné que la jauge de scie (2) peut être enfilée sur la broche intramédullaire (11), puis couplée avec la jauge de rotation (1), et étant donné que la broche intramédullaire (11) forme un axe de pivotement pour la jauge de scie (2), axe autour duquel cette dernière peut pivoter dans une plage de ±10° par rapport à l'horizontale,
- un mécanisme d'entraînement (14) qui fait pivoter la jauge de scie (2) autour de l'axe de pivotement prédéfini par la broche intramédullaire (11) dans la section horizontale (H) du fémur, et
- une branche (7) orientée vers le fémur et qui peut être couplée de manière amovible avec l'unité fonctionnelle se composant de la jauge de scie (2) et de la jauge de rotation (1), branche qui présente à son extrémité une sonde de contact fémoral (6) ventrale sous forme de tourillon (8) vertical situé sur la branche (7) pour l'appui ventral sur le fémur (13).

2. Système selon la revendication 1, dans lequel la broche intramédullaire (1) est articulée sur la jauge de rotation (1) au moyen d'un ajustement à bille à frottement dur.

3. Système selon la revendication 1 ou 2, dans lequel le mécanisme d'entraînement (14) de la jauge de rotation peut être réglé progressivement.

4. Système selon la revendication 3, dans lequel le mécanisme d'entraînement (14) s'encliquette à des positions de réglage prédéfinies.

5. Système selon l'une quelconque des revendications 1 à 4, dans lequel la jauge de scie (2) présente des moyens de fixation (20) pour la fixation temporaire sur le fémur.
